# EUROPEAN PATENT SPECIFICATION

(11) **EP 1 571 999 B1**
(45) Date of publication and mention of the grant of the patent: **28.11.2007**
(21) Application number: 03775713.5
(22) Date of filing: 04.12.2003
(51) Int. Cl.: A61B 6/08

(54) **METHOD OF TOMOGRAPHIC IMAGING**
TOMOGRAPHISCHIES BILDGEBUNGSVERFAHREN
PROCEDE D'IMAGERIE TOMOGRAPHIQUE

(30) Priority: 11.12.2002 EP 02102718
(43) Date of publication of application: 14.09.2005
(73) Proprietor: Philips Intellectual Property & Standards GmbH, 20099 Hamburg (DE); Koninklijke Philips Electronics N.V., 5621 BA Eindhoven (NL)
(72) Inventor: NETSCH, Thomas, c/o Philips Intellectual, 52066 Aachen (DE); BARSCHDORF, Hans, c/o Philips Intellectual, 52066 Aachen (DE)
(74) Representative: Volmer, Georg
(86) International application number: PCT/IB2003/005741
(87) International publication number: WO 2004/052206

(56) References cited:
- WO-A-98/02091
- WO-A-02/091924
- US-A- 5 673 300
- US-B1- 6 195 409

## Description

The invention relates to a method of tomographic imaging, and in particular a CT or MR method, for the repetitive production of diagnostic slice images of a part of a patient's body, having the following method steps:
a) making of current reference slice images of the part of the body,
b) determination of a geometrical transformation by which the current reference slice images are brought into agreement with earlier reference slice images of the part of the body,
c) calculation of current imaging parameters by transforming earlier imaging parameters by means of the geometrical transformation determined in step b),
d) making of a current diagnostic slice image, the position and orientation in three dimensions of the image plane of the diagnostic slice image being determined by the current imaging parameters calculated in step c).

The invention also relates to a computer program and to a tomographic imaging unit having image-making means for performing the method.

Tomographic imaging methods, and particularly CT and MR methods, have proved to be powerful tools in the armory of modern-day medical diagnosis.

To enable pathological fmdings, such as advancing tumorous conditions for example, to be examined over fairly lengthy periods of time, it is necessary for diagnostic slice images of a part of a patient's body to be made repetitively at different times. Due to the different contrast characteristics of different imaging methods of examination, there are even diagnostic advantages to be drawn from slice images of an object for examination that are made under different modalities.

When diagnostic slice images are made as a repetitive process, it is crucial that the position and orientation in three dimensions, relative to the part of the patient's body that is being examined, of the slice images made at different times or under different modalities should agree as closely as possible so that, for example, the advance of the condition can be accurately observed. For this purpose, it is usual for reference slice images of the part of the body to be made before the actual diagnostic slice image is made. By calculating a geometrical transformation, it is possible for a fresh, current reference slice image that is made to be brought into congruence with earlier reference slice images. The method that is required for this purpose is a method of optimization in which the sets of image data for the reference slice images made at different times are brought into agreement. From the geometrical transformation that is calculated, transformation parameters are obtained that are taken as a basis for calculating current imaging parameters. For diagnostic slice images, the current imaging parameters are then used to enable the image planes of the diagnostic slice images to be set repeatably (see for example J.M. Fitzpatrick, D.L. Hill and C.R. Maurer Jr.: "Chapter 8: Image Registration" in M. Sonka and J.M. Fitzpatrick (eds.) "Handbook of Medical Imaging, Volume 2: Medical Image Processing and Analysis", pages 447-513, SPIE Press, Bellingham WA, 2000; J.B.Maintz and M.A. Viergever: "A Survey of Medical Image Registration", Medical Image Analysis, Vol. 2(1), pages 1-36, 1998).

It is a disadvantage of the known methods that the accuracy obtained in calculating the imaging parameters is often not high enough. A particular reason for this is that the accuracy with which the geometrical transformation is determined is very much dependent on the presetting of the image planes of the reference slice images, because the resolution of the image in the image plane that is preset is, as a rule, considerably greater than in a direction perpendicular thereto. Because of the time required to make the reference slice images, it is not possible, and this is a disadvantage, for image data to be acquired, as part of the making of the reference slice images, with adequate resolution in all three dimensions. What would be needed for this purpose would be high-resolution volumetric imaging, something that is not possible in practice.

Taking the above as a point of departure, it is an object of the present invention to provide an improved method of tomographic imaging that allows accurate calculation of the imaging parameters required for the making of the diagnostic slice image and that manages with only a minimal image-making time for the making of the reference slice images.

In accordance with the invention, this object is achieved by a method of tomographic imaging of the kind mentioned in the opening paragraph in which there are made, in step a) of the method, at least two current reference slice images whose image planes are preset in such a way that their relative position and orientation in three dimensions agree with the relative position and orientation in three dimensions of the earlier reference slice images, and in which the geometrical transformation is determined in step b) in such a way that, by it, all the current reference slice images are brought into agreement with the corresponding earlier reference slice images simultaneously.

The finding on which the invention is based is that it is advantageous if, for calculating the imaging parameters, sets of image data from a plurality of current and earlier reference slice images, in which the relative positions and orientations in three dimensions of the image planes are preset at fixed values and are always the same, are brought into agreement simultaneously by means of a single geometrical transformation. By using a set comprising a plurality of reference slice images whose image planes are, if possible, differently oriented to determine the geometrical transformation, the image resolution that is actually available in this case in different directions in space is increased, but without it being necessary for any time-consuming high-resolution volumetric imaging having to be performed for the making of the reference slice images. Overall, there is an increase in accordance with the invention in the field of view (FOV) covered by the reference slice images as compared with methods known from the prior art, which has a beneficial effect on the accuracy and in particular on the unambiguousness with which rotations and translations of the part of body being examined are detected. In accordance with the invention, a plurality of reference slice images, whose individual resolution may be comparatively low, can be made in a short time. What is achieved by the method according to the invention is that low image resolution in a reference slice image in one direction of space is compensated for by correspondingly higher image resolution in another reference slice image in this same direction in space.

In the method according to the invention, the geometrical transformation may, for example, be determined in step b) of the method by identifying reference points in the current reference slice images that agree with corresponding reference points in the earlier reference slice images. By the finding of reference points that agree, regions of the image are defined at whose centers the reference points are respectively situated. The geometrical transformation is then the result of converting the coordinates of the reference points in the earlier reference slice images into the coordinates of the reference points identified in the current reference slice images. In this variant of the method according to the invention, the reference points that are to be converted into one another may, for example, be identified manually by a user, which he does by comparing the earlier and current reference slice images, which are displayed for this purpose on a suitable output unit, with one another and, in the course of this, selecting the appropriate points in the reference slice images interactively.

In the method according to the invention, it is found to be advantageous if the geometrical transformation determined in step b) of the method is a rigid or an affine transformation that is defined by a corresponding set of transformation parameters. Rigid transformations, being a special case of affine transformations, define turning movements and displacements, i.e. rotations and translations, whereas affine transformations map points to points, straight lines to straight lines and planes to planes, in which case parallelism and relative lengths are maintained. Hence, it is possible with the rigid transformations to sense, for example, shifts in the position of the head of a patient being examined when the making of a diagnostic slice image is repeated.

In a variant of the method according to the invention that is an alternative or addition to the manual procedure described above for determining the geometrical transformation, the set of transformation parameters may be determined automatically by, by means of a suitable algorithm, optimizing a measure of similarity that represents the similarity of the current reference slice images to the corresponding earlier ones. To enable the set of transformation parameters by which the reference slice images are brought into agreement to be determined automatically, it is necessary for the similarity of the geometrically transformed current reference slice images to the corresponding earlier reference slice images to be quantifiable. What are suitable for this purpose are known measures of similarity such as, for example, the sum of the squares of the differences in the gray values of image points (SSD - sum of squares of differences) or the correlation coefficient (CC) or even measures of similarity used in information theory (e.g. MI - mutual information). The optimizing algorithms concerned are conventional algorithms such as, for example, the Gauss Newton or Downhill Simplex algorithm.

A plurality of parallel reference slice images in each of the head-foot, anterior-posterior and right-left directions are preferably made in step a) of the method, the image resolution being selected to be higher in the image planes than perpendicularly thereto. Hence, in accordance with the invention, low image resolution in the head-foot direction, for example, may be compensated for by high image resolutions in the other directions mentioned. It is advantageous that, in this way, only comparatively short imaging times are required for making the reference slice images.

A computer program as detailed in claim 5 is suitable for performing the method according to the invention, on for example a computer connected to a tomographic imaging unit. The software required for this purpose may advantageously be made available to users of tomographic imaging units on a suitable data carrier, such as a floppy disk or a CD-ROM, or over a data network (the internet) for downloading.

A tomographic imaging unit for performing the method according to the invention is the subject of claim 6, under which a computer is so set up in respect of software that the making of the diagnostic slice images takes place by the method described above.

These and other aspects of the invention are apparent from and will be elucidated with reference to the embodiments described hereinafter.

In the drawings:
Fig. 1 is a diagrammatic representation of the method of tomographic imaging according to the invention.
Fig. 2 is a view of reference slice images in the method of tomographic imaging according to the invention.

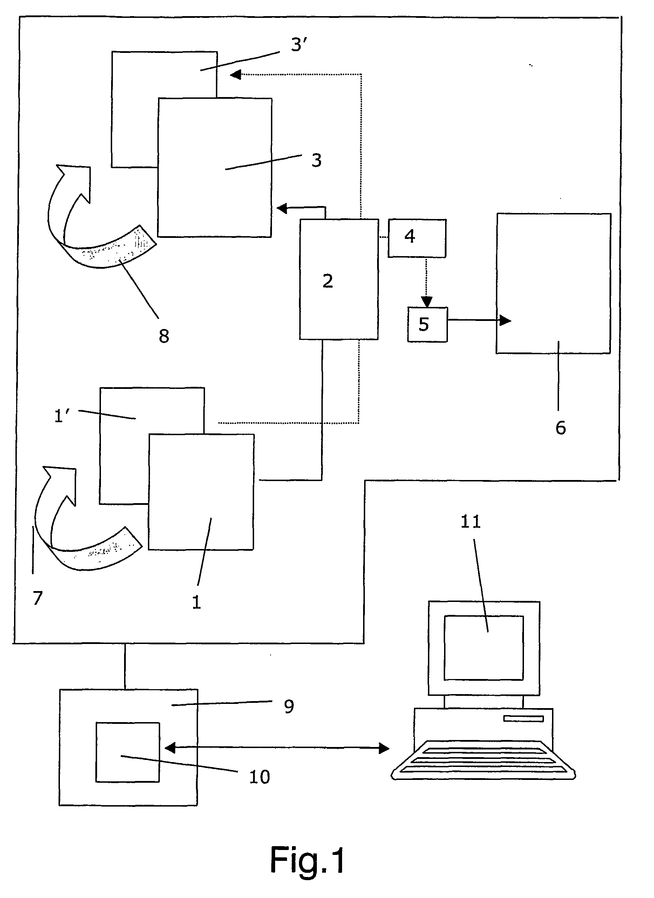

Fig. 1 is a diagram showing the making of a current reference slice image 1 of a part of a patient's body, which image is produced in particular by the MR or CT method of imaging. To allow a geometrical transformation 2 to be determined, an earlier reference slice image 3 is brought into agreement with the current reference slice image 1. From a set 4 of transformation parameters for the geometrical transformation 2, current imaging parameters 5 are then calculated, which are then used to set the position and orientation of the image plane of a diagnostic slice image 6.

It is ensured in this way that, as far as possible, the positions and orientations in three dimensions, relative to the part of the body being examined, of the diagnostic slice images made at different times or under different modalities agree.

In accordance with the invention, at least two current reference slice images 1, 1' are made. The relative positions and orientations, as symbolized by the arrow 7, of the reference slice images 1 and 1' agree in this case with the relative positions and orientations 8 of the corresponding earlier reference slice images 3, 3'. In a further step of the method according to the invention, the geometrical transformation 2 is determined in such a way that both of the current reference slice images 1, 1' are brought into agreement with the corresponding earlier reference slice images 3, 3' simultaneously. The set 4 of transformation parameters is determined automatically in this case by causing a measure of similarity that represents the similarity of the current reference slice images 1 and 1' to the earlier reference slice images 3 and 3' corresponding thereto to be optimized by means of a suitable algorithm. The current imaging parameters 5 are then calculated on this basis.

The method according to the invention may be performed by means of a tomographic imaging unit 9 that has image-making means 10. The image-making means 10 make the reference slice images 1, 1', 3, 3' and the diagnostic slice image 6, with a computer 11 belonging to the tomographic imaging unit operating the image-making means 10 and calculating the imaging parameters 5 automatically by the method described above.

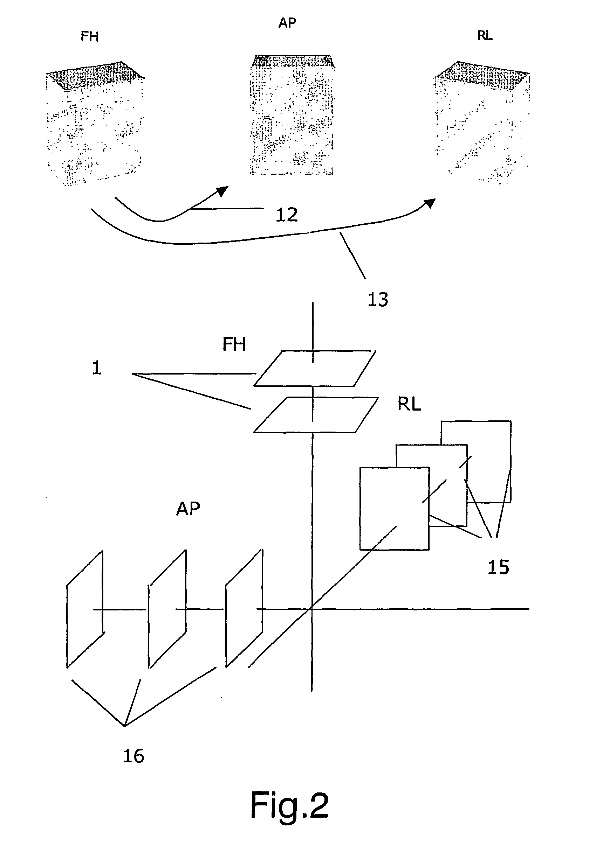

Fig. 2 shows that, for the making of reference slice images, the user of a tomographic imaging unit has available to him the foot-head (FH), anterior-posterior (AP) and right-left (RL) directions, in which the positions and orientations 12, 13 in three dimensions are different, in which case a plurality of parallel reference slice images 14, 15, 16 (so-called stacks) can be made in any of the directions mentioned.

By the method described above, current and earlier stacks of reference slice images can be used to calculate current imaging parameters.

## Claims

1. A method of tomographic imaging, and particularly a CT or MR method, for repetitively producing diagnostic slice images of a part of a patient's body, having the following method steps:
a) making of current reference slice images (1) of the part of the body,
b) determination of a geometrical transformation (2) by which the current reference slice images (1) are brought into agreement with earlier reference slice images (3) of the part of the body,
c) calculation of current imaging parameters (5) by transforming earlier imaging parameters by means of the geometrical transformation (2) determined in step b),
d) making of a current diagnostic slice image (6), the position and orientation in three dimensions of the image plane of the diagnostic slice image (6) being determined by the current imaging parameters (5) calculated in step c),
**characterized in that** there are made in step a) of the method at least two current reference slice images (1, 1') whose image planes are preset in such a way that their relative positions and orientations (7) in three dimensions agree with the relative positions and orientations (8) in three dimensions of the earlier reference slice images (3, 3'), and **in that** the geometrical transformation (2) is determined in step b) in such a way that, by it, all the current reference slice images are brought into agreement with the corresponding earlier reference slice images simultaneously.

2. A method as claimed in claim 1, **characterized in that** the geometrical transformation (2) is determined in step b) of the method by identifying reference points in the current reference slice images (1, 1') that agree with corresponding reference points in the earlier reference slice images (3, 3').

3. A method as claimed in claim 1, **characterized in that** the geometrical transformation (2) determined in step b) of the method is a rigid or an affine transformation that is defined by a set (4) of transformation parameters, the set (4) of transformation parameters being determined automatically by, by means of a suitable algorithm, optimizing a measure of similarity that represents the similarity of the current reference slice images (1, 1') to the corresponding earlier ones (3, 3').

4. A method as claimed in claim 1, **characterized in that** a plurality of parallel reference slice images (14, 15, 16) are made in each of the head-foot, anterior-posterior and right-left directions in step b) of the method, the image resolution being selected to be higher in the image planes than perpendicularly thereto.

5. A computer program for performing the method claimed in claim 1, which automatically determines imaging parameters by which the position and orientation in three dimensions of the image plane of a diagnostic slice image (6) are determined, so doing by
a) receiving current image data for current reference slice images (1) and earlier image data for earlier reference slice images (3) as an input,
b) determining a geometrical transformation (2) by which the current image data is brought into agreement with the earlier image data,
c) calculating the current imaging parameters (5) by transforming earlier imaging parameters by the geometrical transformation (2) determined in step b),
**characterized in that** the input in step a) comprises current and earlier image data for, in each case, at least two current (1, 1') and earlier reference slice images (3, 3'), and **in that**, in step b), the geometrical transformation (2) brings the image date for all the current reference slice images (1, 1') into agreement with the image data for the corresponding earlier reference slice images (3, 3') simultaneously, a set (4) of transformation parameters defining the geometrical transformation being determined by, by means of a suitable optimizing algorithm, maximizing a measure of similarity that represents the similarity of the current image data to the corresponding earlier image data.

6. A tomographic imaging unit (9) having image-making means (10) that make diagnostic slice images (6), and having a computer (11) that operates the image-making means (10) and for this purpose calculates imaging parameters (5) that determine the particular positions and orientations in three dimensions of the image planes of the diagnostic slice images (6), **characterized in that** the computer (11) is so set up in respect of software that the making of the diagnostic slice images (6') takes place by the method claimed in claim 1.

## Patentansprüche

1. Tomographisches Bildgebungsverfahren, insbesondere CT- oder MR-Verfahren, zur wiederholten Erzeugung von diagnostischen Schichtbildern eines Körperteils eines Patienten, mit den folgenden Verfahrensschritten:
a) Aufnahme von aktuellen Referenz-Schichtbildem (1) des Körperteils,
b) Bestimmung einer geometrischen Transformation (2), durch welche die aktuellen Referenz-Schichtbilder (1) mit früheren Referenz-Schichtbildem (3) des Körperteils in Übereinstimmung gebracht werden,
c) Berechnung von aktuellen Bildgebungsparametem (5), indem frühere Bildgebungsparameter gemäß der in Schritt b) bestimmten geometrischen Transformation (2) transformiert werden,
d) Erzeugung eines aktuellen diagnostischen Schichtbildes (6), wobei die räumliche Lage und Orientierung der Bildebene des diagnostischen Schichtbildes (6) durch die in Schritt c) berechneten aktuellen Bildgebungsparameter (5) bestimmt wird,
**dadurch gekennzeichnet, dass** im Verfahrensschritt a) wenigstens zwei aktuelle Referenz-Schichtbilder (1,1') aufgenommen werden, deren Bildebenen derart vorgegeben werden, dass deren relative räumliche Lage und Orientierung (7) in den drei Raumrichtungen mit der relativen räumlichen Lage und Orientierung (8) der früheren Referenz-Schichtbilder (3,3') in den drei Raumrichtungen übereinstimmt, und dass in Schritt b) die geometrische Transformation (2) derart bestimmt wird, dass dadurch gleichzeitig alle aktuellen Referenz-Schichtbilder mit den korrespondierenden früheren Referenz-Schichtbildem in Übereinstimmung gebracht werden.

2. Verfahren nach Anspruch 1, **dadurch gekennzeichnet, dass** im Verfahrensschritt b) die geometrische Transformation (2) bestimmt wird, indem in den aktuellen Referenz-Schichtbildern (1,1') Referenzpunkte identifiziert werden, welche mit entsprechenden Referenzpunkten in den früheren Referenz-Schichtbildem (3, 3') übereinstimmen.

3. Verfahren nach Anspruch 1, **dadurch gekennzeichnet, dass** es sich bei der im Verfahrensschritt b) bestimmten geometrischen Transformation (2) um eine starre oder um eine affine Transformation handelt, welche durch einen Transformations-Parametersatz (4) beschrieben wird, wobei der Transformations-Parametersatz (4) automatisch bestimmt wird, indem ein die Ähnlichkeit der aktuellen (1, 1') mit den korrespondierenden früheren Referenz-Schichtbildem (3, 3') wiedergebendes Ähnlichkeitsmaß mittels eines geeigneten Algorithmus optimiert wird.

4. Verfahren nach Anspruch 1, **dadurch gekennzeichnet, dass** im Verfahrensschritt b) jeweils mehrere parallele Referenz-Schichtbilder (14,15,16) in Kopf-Fuß-, Anteriori-Posteriori- und Rechts-Links-Richtung aufgenommen werden, wobei die Bildauflösung innerhalb der Bildebenen höher gewählt wird als senkrecht dazu.

5. Computerprogramm zur Durchführung des Verfahrens nach Anspruch 1, welches Bildgebungsparameter automatisch ermittelt, durch welche die räumliche Lage und Orientierung der Bildebene eines diagnostischen Schichtbildes (6) bestimmt werden, indem es
a) aktuelle Bilddaten von aktuellen Referenz-Schichtbildem (1) und frühere Bilddaten von früheren Referenz-Schichtbildem (3) als Eingabe erhält,
b) eine geometrische Transformation (2) bestimmt, durch welche die aktuellen Bilddaten mit den früheren Bilddaten in Übereinstimmung gebracht werden,
c) die aktuellen Bildgebungsparameter (5) berechnet, indem es frühere Bildgebungsparameter gemäß der in Schritt b) bestimmten geometrischen Transformation (2) transformiert,
**dadurch gekennzeichnet, dass** die Eingabe in Schritt a) aktuelle und frühere Bilddaten von jeweils wenigstens zwei aktuellen (1,1') bzw. früheren Referenz-Schichtbildem (3,3') umfasst und dass in Schritt b) durch die geometrische Transformation (2) gleichzeitig die Bilddaten aller aktuellen Referenz-Schichtbilder (1,1') mit den korrespondierenden Bilddaten der früheren Referenz-Schichtbilder (3,3') in Übereinstimmung gebracht werden, wobei ein die geometrische Transformation beschreibender Transformations-Parametersatz (4) ermittelt wird, indem ein die Ähnlichkeit der aktuellen mit den korrespondierenden früheren Bilddaten wiedergebendes Ähnlichkeitsmaß mittels eines geeigneten Optimierungsalgorithmus maximiert wird.

6. Tomographisches Bildgebungsgerät (9) mit Bildaufnahme-Mitteln (10), die diagnostische Schichtbilder (6) aufnehmen, und mit einem Computer (11), der die Bildaufnahme-Mittel (10) ansteuert und dazu Bildgebungsparameter (5) berechnet, welche die jeweilige räumliche Lage und Orientierung der Bildebenen der diagnostischen Schichtbilder (6) bestimmen, **dadurch gekennzeichnet, dass** der Computer (11) programmtechnisch derart eingerichtet ist, dass die Aufnahme der diagnostischen Schichtbilder (6') gemäß dem Verfahren nach Anspruch 1 erfolgt.

## Revendications

1. Procédé d'imagerie tomographique et en particulier un procédé CT ou MR pour la production répétitive d'images diagnostiques de tranche d'une partie du corps d'un patient, le procédé ayant les étapes suivantes consistant à:
a) faire des images courantes de tranche de référence (1) de la partie du corps,
b) déterminer une transformation géométrique (2) par laquelle les images courantes de tranche de référence (1) sont mises en concordance avec des images antérieures de tranche de référence (3) de la partie du corps,
c) calculer des paramètres courants d'imagerie (5) en transformant des paramètres antérieurs d'imagerie au moyen de la transformation géométrique (2) qui est déterminée dans l'étape b),
d) faire une image diagnostique courante de tranche (6), la position et l'orientation en trois dimensions du plan d'image de l'image diagnostique de tranche (6) étant déterminées par les paramètres courants d'imagerie (5) qui sont calculés dans l'étape c),
**caractérisé en ce que** l'on fait, dans l'étape a) du procédé, au moins deux images courantes de tranche de référence (1, 1') dont les plans d'image sont préréglés de telle façon que leurs positions et leurs orientations relatives (7) en trois dimensions s'accordent avec les positions et les orientations relatives (8) en trois dimensions des images antérieures de tranche de référence (3, 3') et **en ce que** l'on détermine la transformation géométrique (2) dans l'étape b) de telle façon que, par elle, toutes les images courantes de tranche de référence soient mises simultanément en concordance avec les images antérieures correspondantes de tranche de référence.

2. Procédé selon la revendication 1, **caractérisé en ce que** l'on détermine la transformation géométrique (2) dans l'étape b) du procédé en identifiant des points de référence dans les images courantes de tranche de référence (1, 1') qui s'accordent avec les points de référence correspondants dans les images antérieures de tranche de référence (3, 3').

3. Procédé selon la revendication 1, **caractérisé en ce que** la transformation géométrique (2) qui est déterminée dans l'étape b) du procédé est une transformation rigide ou affine qui est définie par un ensemble (4) de paramètres de transformation, l'ensemble (4) de paramètres de transformation étant déterminé automatiquement par optimisation, au moyen d'un algorithme approprié, d'une mesure de similitude qui représente la similitude des images courantes de tranche de référence (1, 1') aux images antérieures correspondantes (3, 3').

4. Procédé selon la revendication 1, **caractérisé en ce qu'**une pluralité d'images parallèles de tranche de référence (14, 15, 16) sont faites dans chacune des directions de tête à pied, des directions antérieures et postérieures et des directions de droite à gauche dans l'étape b) du procédé, la résolution d'image étant sélectionnée de manière à être plus haute dans les plans d'image que perpendiculairement à ceux-ci.

5. Programme informatique pour mettre en oeuvre le procédé selon la revendication 1, qui détermine automatiquement les paramètres d'imagerie par lesquels la position et l'orientation en trois dimensions du plan d'image d'une image diagnostique de tranche (6) sont déterminées, ce faisant en:
a) recevant, en tant qu'une entrée, les données courantes d'image pour les images courantes de tranche de référence (1) et les données antérieures d'image pour les images antérieures de tranche de référence (3),
b) déterminant une transformation géométrique (2) par laquelle les données courantes d'image sont mises en concordance avec les données antérieures d'image,
c) calculant les paramètres courants d'imagerie (5) en transformant les paramètres antérieurs d'imagerie par la transformation géométrique (2) qui est déterminée dans l'étape b),
**caractérisé en ce que** l'entrée dans l'étape a) comprend les données courantes et antérieures d'image pour, dans chaque cas, au moins deux images courantes (1, 1') et antérieures (3, 3') de tranche de référence et **en ce que**, dans l'étape b), la transformation géométrique (2) met simultanément les données d'image pour toutes les images courantes de tranche de référence (1, 1') en concordance avec les données d'image pour les images antérieures correspondantes de tranche de référence (3, 3'), un ensemble (4) de paramètres de transformation définissant la transformation géométrique étant déterminé par maximisation, au moyen d'un algorithme approprié d'optimisation, d'une mesure de similitude qui représente la similitude des données courantes d'image aux données antérieures correspondantes d'image.

6. Unité d'imagerie tomographique (9) ayant des moyens de fabrication d'image (10) qui font les images diagnostiques de tranche (6) et ayant un ordinateur (11) qui actionne les moyens de fabrication d'image (10) et qui calcule à cette fin les paramètres d'imagerie (5) qui déterminent les positions et les orientations particulières en trois dimensions des plans d'image des images diagnostiques de tranche (6), **caractérisée en ce que** l'ordinateur (11) est réglé, pour ce qui est du logiciel, de telle façon que la fabrication des images diagnostiques de tranche (6') s'effectue par le procédé qui est revendiqué dans la revendication 1.
